(19) Europäisches Patentamt
European Patent Office
Office européen des brevets

(11) Veröffentlichungsnummer: **0 634 923 B1**

(12) # EUROPÄISCHE PATENTSCHRIFT

(45) Veröffentlichungstag der Patentschrift: **20.12.95**

(51) Int. Cl.$^6$: **A61K 7/13**, C09B 57/00

(21) Anmeldenummer: **93907845.7**

(22) Anmeldetag: **29.03.93**

(86) Internationale Anmeldenummer:
**PCT/EP93/00755**

(87) Internationale Veröffentlichungsnummer:
**WO 93/19725 (14.10.93 93/25)**

(54) **MITTEL ZUM FÄRBEN VON KERATINHALTIGEN FASERN**

(30) Priorität: **06.04.92 DE 4211450**
**23.01.93 DE 4301818**

(43) Veröffentlichungstag der Anmeldung:
**25.01.95 Patentblatt 95/04**

(45) Bekanntmachung des Hinweises auf die Patenterteilung:
**20.12.95 Patentblatt 95/51**

(84) Benannte Vertragsstaaten:
**AT BE CH DE DK ES FR GB GR IE IT LI NL PT SE**

(56) Entgegenhaltungen:
**DE-A- 2 716 671**
**FR-A- 2 252 841**
**GB-A- 2 181 750**

**DATABASE WPIL Week 4389, Derwent Publications Ltd., London, GB; AN 89-314090**

(73) Patentinhaber: **Henkel Kommanditgesellschaft auf Aktien**

**D-40191 Düsseldorf (DE)**

(72) Erfinder: **HÖFFKES, Horst**
**Carlo-Schmid-Strasse 113**
**D-40595 Düsseldorf (DE)**
Erfinder: **MÖLLER, Hinrich**
**Haydnstrasse 27**
**D-4019 Monheim (DE)**
Erfinder: **BÜTTNER, Roswitha**
**Kleverstrasse 46**
**D-4000 Düsseldorf (DE)**

**EP 0 634 923 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Gegenstand der Erfindung sind Isatine und Aminosäuren oder Oligopeptide enthaltende Mittel zum Färben von keratinhaltigen Fasern, insbesondere menschlichem Haar. In einer besonderen Ausgestaltung enthalten diese Färbemittel Ammonium- oder Metallsalze.

Für das Färben von keratinhaltigen Fasern, z. B. Haaren, Wolle oder Pelzen, kommen im allgemeinen entweder direktziehende Farbstoffe oder Oxidationsfarbstoffe, die durch oxidative Kupplung einer oder mehrerer Entwicklerkomponenten untereinander oder mit einer oder mehreren Kupplerkomponenten entstehen, zur Anwendung. Mit Oxidationsfarbstoffen lassen sich zwar farbintensive Färbungen mit guten Echtheitseigenschaften erzielen, die Entwicklung der Farbe geschieht jedoch unter dem Einfluß von Oxidationsmitteln wie z. B. $H_2O_2$, was häufig eine Schädigung der Faser zur Folge hat. Direktziehende Farbstoffe werden unter schonenderen Bedingungen appliziert. Ihr Nachteil liegt jedoch darin, daß die Färbungen häufig nur über unzureichende Echtheitseigenschaften verfügen.

Färbesysteme auf Basis von Isatin oder Isatinderivaten bieten hier eine Alternative. Isatin ist als Direktfarbstoff zum Färben von Keratinfasern alleine oder in Kombination mit Chinonfarbstoffen in der deutschen Offenlegungsschrift DE 36 35 147 beschrieben worden. Die Variationsbreite der erzielbaren Nuancen ist jedoch beschränkt. In den allermeisten Fällen erhält man eine goldfarbene Färbung.

Ein anderes Färbeverfahren für keratinische Fasern wird in EP-A 359 465 beschrieben. Hier wird die Färbung mit Hilfe eines aus der Reaktion eines Isatinderivats mit einem Anilinderivat entstehenden Ketimins erzielt. Das Ketimin wird entweder als solches auf keratinische Fasern aufgebracht und entwickelt dort eine Färbung, oder aber eine aus einem Isatinderivat und einem Anilinderivat bestehende Mischung wird auf die Faser aufgebracht und bildet zunächst "in situ" das Ketimin, woraufhin sich auf der Faser die Färbung entwickelt.

Die Färbung wird in beiden Fällen ohne Zusatz von Oxidationsmitteln erzielt. Der katalytische Einfluß des Keratins ist jedoch zur Ausbildung der Färbung zwingend notwendig. Ein weiterer Nachteil ist, daß Anilinderivate dermatologisch und toxikologisch nicht unbedenklich sind.

Aufgabe der vorliegenden Erfindung ist es, ein Färbemittel auf der Grundlage von Isatinderivaten und toxikologisch unbedenklichen aminhaltigen Verbindungen zu finden, wobei auf die Verwendung von Anilinderivaten verzichtet werden soll.

Überraschenderweise wurde nun gefunden, daß Aminosäuren und Oligopeptide mit Isatinderivaten in Gegenwart von keratinhaltigem Material Farbstoffe bilden, die gut auf keratinhaltige Fasern aufziehen.

Gegenstand der Erfindung sind deshalb Mittel zum Färben von keratinhaltigen Fasern, enthaltend mindestens ein Isatinderivat der Formel I

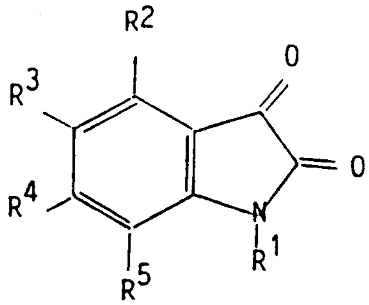

(I),

wobei $R^1$ Wasserstoff, eine Alkylgruppe mit 1 bis 4 C-Atomen, eine Hydroxyalkylgruppe mit 2 bis 4 C-Atomen, eine $C_2$-$C_{20}$-Acylgruppe, eine Phenylgruppe oder eine Benzoylgruppe und $R^2$, $R^3$, $R^4$ und $R^5$ unabhängig voneinander Wasserstoff, Hydroxy, Halogen, Nitrogruppen, Alkylgruppen mit 1 bis 4 C-Atomen, Alkoxygruppen mit 1 bis 4 C-Atomen oder $R^6R^7N$-Gruppen bedeuten, worin $R^6$ und $R^7$ unabhängig voneinander Wasserstoff, Alkylgruppen mit 1 bis 4 C-Atomen oder Hydroxyalkylgruppen mit 2 bis 4 C-Atomen darstellen, und zwei benachbarte Gruppen $R^2$, $R^3$, $R^4$ und $R^5$ auch eine Alkylendioxygruppe mit 1 bis 4 C-Atomen darstellen können, und mindestens eine Aminosäure oder ein aus 2 bis 9 Aminosäuren aufgebautes wasserlösliches Oligopeptid in einem wasserhaltigen Träger.

Als keratinhaltige Fasern kommen z. B. Wolle, Pelze, Felle und menschliche Haare in Betracht. Obwohl die besten Färbungen an Keratinfasern erzielt werden, können die erfindungsgemäßen Färbemittel prinzipiell auch zum Färben anderer Naturfasern wie z. B. Baumwolle, Jute, Sisal, Leinen oder Seide, modifizierter Naturfasern wie z. B. Regeneratcellulose, Nitro-, Alkyl-, Hydroxyalkyl- oder Acetylcellulose und synthetischer Fasern wie z. B. Polyamid-, Polyacrylnitril-, Polyurethan- und Polyesterfasern verwendet werden.

Besonders gute Färbeergebnisse erzielt man mit den erfindungsgemäßen Mitteln, wenn im Isatinderivat der Formel I $R^1$ Wasserstoff ist und $R^2$, $R^3$, $R^4$ und $R^5$ unabhängig voneinander Wasserstoff, Hydroxy, Halogen, Nitrogruppen oder $R^6R^7N$-Gruppen darstellen, worin $R^6$ und $R^7$ Wasserstoff bedeuten. Am vorteilhaftesten ist der Grundkörper Isatin selbst.

Als Aminosäuren kommen alle natürlich vorkommenden und synthetischen Aminosäuren in Frage, z. B. Arginin, Cystein, Methionin, Prolin, Tyrosin, Valin, Glycin, Glutaminsäure, Histidin, Asparaginsäure, Alanin, Tryptophan, Cystin, Lysin, Hydroxyprolin, Leucin, Isoleucin, Phenylalanin, Serin, Threonin, Ornithin, Asparagin, Glutamin, 3-Aminopropionsäure, 6-Aminocapronsäure und deren Gemische, die z. B. durch Hydrolyse aus pflanzlichen oder tierischen Proteinen zugänglich sind.

Dabei spielt es keine Rolle, ob die natürlichen L-Aminosäuren oder ein Gemisch aus D- und L-Aminosäuren verwendet werden; es können auch D-Aminosäuren verwendet werden. Es muß auch nicht zwischen sauer und alkalisch reagierenden Aminosäuren entschieden werden.

Vorzugsweise ist in den erfindungsgemäßen Färbemitteln neben einem Isatinderivat der Formel I jedoch mindestens eine der $\alpha$-Aminosäuren enthalten, vorzugsweise aus der Gruppe Arginin, Cystein, Methionin, Prolin, Tyrosin, Valin, Glycin, Glutaminsäure, Histidin, Asparaginsäure, Alanin, Tryptophan, Cystin, Lysin, Hydroxyprolin, Leucin, Isoleucin, Phenylalanin, Serin, Ornithin und Threonin enthalten.

Geeignete Oligopeptide sind alle aus natürlich vorkommenden und synthetischen Aminosäuren aufgebauten Oligopeptide. Die Oligopeptide können dabei natürlich vorkommende oder synthetische Oligopeptide, aber auch die in Polypeptid- oder Proteinhydrolysaten enthaltenen Oligopeptide sein, sofern sie über eine für die Anwendung in den Färbemitteln ausreichende Wasserlöslichkeit verfügen. Ohne Einschränkung sind z. B. zu nennen: Glutathion oder die in den Hydrolysaten von Collagen, Keratin, Casein, Elastin, Sojaprotein, Weizengluten oder Mandelprotein enthaltenen Oligopeptide.

Mit den zur Verfügung stehenden Aminosäuren und Oligopeptiden läßt sich ein breites Farbspektrum abdecken. Zur Farbnuancierung kann jedoch auch eine Mischung von verschiedenen Aminosäuren oder Oligopeptiden verwendet werden.

Ein weiterer Erfindungsgegenstand ist deshalb ein Mittel zum Färben von keratinhaltigen Fasern, das neben einem Isatinderivat der Formel I mindestens eine $\alpha$-Aminosäure, vorzugsweise eine aus der oben genannten Gruppe, enthält.

Besonders gute Färbeergebnisse erzielt man, wenn die erfindungsgemäßen Färbemittel zusätzlich bestimmte Ammonium- oder Metallsalze enthalten.

Ein weiterer Erfindungsgegenstand sind Mittel zum Färben von keratinhaltigen Fasern, die zusätzlich mindestens ein Salz ausgewählt aus der Gruppe der Ammonium-, Lithium-, Magnesium-, Calcium-, Strontium-, Barium-, Aluminium-, Titan-, Mangan-, Eisen-, Kobalt-, Nickel-, Kupfer-, Silber-, Zink-, Lanthan-, Cer-, Praseodym-, Neodym- und Gadoliniumsalze enthalten.

Die Salze sollten in den zum Einsatz kommenden Mengen physiologisch verträglich sein.

Ein weiterer Erfindungsgegenstand sind deshalb Mittel zum Färben von keratinhaltigen Fasern, wobei als Salze die Halogenide, Nitrate, Nitrite, Acetate, Lactate, Glykolate, Tartrate, Citrate, Gluconate, Propionate, Sulfate, Carbonate, Succinate, Phosphate, Phosphonate oder Sulfonate von Ammoniumionen bzw. der oben aufgeführten Metalle enthalten sind.

Besonders bevorzugt sind Färbemittel, die Salze ausgewählt aus der Gruppe Ammoniumcarbonat, -chlorid und -acetat, Lithiumbromid, Magnesiumchlorid und -sulfat, Calciumchlorid und -gluconat, Strontiumchlorid und -nitrat, Aluminiumchlorid und -lactat, Zinkchlorid und -sulfat, Kupfer-(II)-chlorid und -sulfat und Lanthannitrat enthalten.

Die erfindungsgemäßen Färbemittel ergeben bereits bei physiologisch verträglichen Temperaturen intensive Färbungen mit einem großen Nuancenspektrum. Sie eignen sich deshalb besonders zum Färben von menschlichen Haaren.

Ein weiterer Erfindungsgegenstand sind Haarfärbemittel enthaltend mindestens ein Isatin der Formel I in einer Menge von 0,3 bis 65, vorzugsweise 6 bis 20 mMol, mindestens eine Aminosäure oder ein aus 2 bis 9 Aminosäuren aufgebautes wasserlösliches Oligopeptid in einer Menge von 0,3 bis 65, vorzugsweise 6 bis 20 mMol, jeweils bezogen auf 100 g der gesamten Färbemittelzubereitung, und einen wasserhaltigen Träger.

In einer besonders bevorzugten Ausführungsform enthalten diese Haarfärbemittel mindestens ein Salz ausgewählt aus der Gruppe der Ammonium-, Lithium-, Magnesium-, Calcium-, Strontium-, Barium-, Aluminium-, Titan-, Mangan-, Eisen-, Kobalt-, Nickel-, Kupfer-, Silber-, Zink-, Lanthan-, Cer-, Praseodym-, Neodym- und Gadoliniumsalze, in einer Menge von 0,3 bis 65, vorzugsweise 2 bis 15 mMol, bezogen auf 100 g der gesamten Färbemittelzubereitung, und einen wasserhaltigen Träger.

Geeignete wasserhaltige Träger sind z. B. Cremes, Emulsionen, Gele, oder auch tensidhaltige schäumende Lösungen, wie z. B. Shampoos, oder andere Zubereitungen, die für die Anwendung auf dem Haar geeignet sind.

Übliche Bestandteile solcher Zubereitungen sind z. B. Netz- und Emulgiermittel, wie anionische, nichtionische oder ampholytische Tenside, z. B. Fettalkoholsulfate, Alkansulfonate, $\alpha$-Olefinsulfonate, Fettalkoholpolyglykolethersulfate, Alkylglycoside, Ethylenoxidanlagerungsprodukte an Fettalkohole, an Fettsäuren, an Alkylphenole, an Sorbitanfettsäureester, an Fettsäurepartialglyceride und Fettsäurealkanolamide. Verdikkungsmittel, z. B. Fettalkohole, Fettsäuren, Paraffinöle, Fettsäureester und andere Fettkomponenten in emulgierter Form. Wasserlösliche polymere Verdickungsmittel wie natürliche Gummen, z. B. Gummi arabicum, Karaya-Gummi, Guar-Gummi, Johannisbrotkernmehl, Leinsamengummen und Pektin, biosynthetische Gummen, z. B. Xanthan-Gummi und Dextrane, synthetische Gummen, z. B. Agar-Agar und Algin, Stärke-Fraktionen und -Derivate wie Amylose, Amylopektin und Dextrine, modifizierte Cellulosemoleküle, z. B. Methylcellulose, Hydroxyalkylcellulose und Carboxymethylcellulose, Tone wie z. B. Bentonit oder vollsynthetische Hydrokolloide, z. B. Polyvinylalkohol oder Polyvinylpyrrolidon, haarpflegende Zusätze, wie z. B. wasserlösliche kationische, anionische, amphotere oder zwitterionische Polymere, Pantothensäure, Vitamine, Pflanzenextrakte oder Cholesterin, Elektrolyt- und Puffersalze, pH-Stellmittel, Komplexbildner und Parfumöle sowie Reduktionsmittel zur Stabilisierung der Inhaltsstoffe, z. B. Ascorbinsäure.

Der pH-Wert der Zubereitung liegt entweder im Bereich des sich für die jeweilige Aminosäure, Aminosäuremischung bzw. Oligopeptid oder Oligopeptidmischung spontan einstellenden pH-Wertes, er kann aber auch auf einen Wert zwischen 3 und 10 eingestellt werden, vorzugsweise liegt der pH-Wert bei ca. 6. Dabei ist zu beachten, daß die Farbnuance in einigen Fällen pH-abhängig ist.

Für die Konfektionierung der Ammonium- oder Metallsalz-haltigen Färbemittel gibt es mehrere Möglichkeiten. Die drei Basisbestandteile Isatinderivat, Aminosäure (bzw. Oligopeptid) und Salz können gemeinsam in einer wasserhaltigen Zubereitung oder aber auch in zwei oder drei voneinander getrennten wasserhaltigen Zubereitungen enthalten sein.

In der folgenden Darstellung steht I für Isatinderivat, A für Aminosäure (bzw. Oligopeptid), S für Salz:

1) Konfektionierung in einem Behälter: I + A + S

2) Konfektionierung in zwei Behältern:

a) Behälter 1 : I + S, Behälter 2 : A

b) Behälter 1 : A + S, Behälter 2 : I

c) Behälter 1 : A + I, Behälter 2 : S

Bei jeder der drei Konfektionierungsvarianten 2a), 2b) und 2c) können die Komponenten vor der Anwendung auf dem Haar zusammengemischt werden oder aber nacheinander auf das Haar aufgebracht werden. Bei einer getrennten Applikation auf dem Haar bestehen die beiden Möglichkeiten, zunächst den Inhalt des Behälters 1 und anschließend den Inhalt des Behälters 2 auf das Haar aufzutragen oder aber zuerst den Inhalt des Behälters 2 und anschließend den Inhalt des Behälters 1 aufzutragen.

3) Konfektionierung in drei Behältern:

Behälter 1 : I, Behälter 2 : A, Behälter 3 : S.

Die drei Komponenten I, A und S können in einer beiliebigen Reihenfolge nacheinander auf das Haar aufgebracht werden, sie können aber auch kurz vor der Anwendung zusammengemischt werden.

Eine weitere Möglichkeit besteht darin, zunächst nur zwei der drei Komponenten I, A und S zusammenzumischen, diese Mischung auf das Haar aufzubringen, um dann erst die dritte Komponente hinzuzugeben.

Bevorzugt sind die Konfektionierungen in einem oder in zwei Behältern. Bei einer Konfktionierung in zwei Behältern ist die Variante 2c) bevorzugt, wobei die Haare mit der Salz-Zubereitung vorbehandelt werden.

Die folgenden Beispiele sollen den Erfindungsgegenstand näher erläutern, ohne ihn jedoch hierauf zu beschränken.

## Beispiele

Erfindungsgemäße Färbemittel **ohne** Ammonium- oder Metallsalz

Beispiel 1

Es wurden wäßrige Lösungen bzw. Aufschlämmungen von jeweils 2 Gew.-% Isatin mit 2 Gew.-% $\alpha$-Aminosäure bzw. mit dem Tripeptid Glutathion bereitet und darin zu 90 % ergraute, aber nicht besonders vorbehandelte Haarsträhnen 2 Stunden lang bei 45 °C eingebracht. Es wurden folgende Färbungen erzielt:

Tabelle 1

| Aminosäure | Nuance | pH-Wert |
|---|---|---|
| L-Arginin | rot | 9,2 |
| L-Lysin | rot | 8,9 |
| L-Cystein | orangebraun | 4,2 |
| L-Methionin | orangerot | 4,6 |
| L-Tyronin | goldorange | 3,6 |
| L-Valin | rotorange | 4,7 |
| L-Glycin | orangerot | 5,7 |
| L-Glutaminsäure | orangerot | 3,4 |
| L-Histidin | orangerot | 6,7 |
| L-Asparaginsäure | braunorange | 3,2 |
| L-Alanin | braunorange | 5,6 |
| DL-Tryptophan | goldbraun | 4,8 |
| L-Cystin | goldorange | 4,9 |
| L-Leucin | orangerot | 6,3 |
| L-Isoleucin | orangerot | 5,0 |
| DL-Phenylalanin | goldbraun | 4,9 |
| DL-Serin | orangerot | 5,1 |
| DL-Threonin | orangerot | 4,8 |
| L-Hydroxyprolin | grünblau | 5,8 |
| L-Prolin | blau | 4,6 |
| L-Glutathion | orangerot | 2,6 |

Die in der Tabelle angegebenen pH-Werte wurden gegen Ende der Reaktion des Isatins mit der jeweiligen Aminosäure bzw. dem Glutathion gemessen.

Beispiel 2

Es wurde folgendes Färbemittel zur Anwendung auf dem Haar bereitet:

| | |
|---|---|
| Johannisbrotkernmehl | 2,0 g |
| Natriumacetat | 1,0 g |
| Ascorbinsäure | 1,0 g |
| Fettalkohol-$C_{12}$-$C_{18}$-sulfat, Natriumsalz | 1,0 g |
| Isatin | 2,0 g |
| L-Prolin | 0,5 g |
| DL-Tryptophan | 1,5 g |
| Wasser | ad 100 g |

Das Färbemittel wurde auf hellblondes Naturhaar aufgetragen und dort 2 Stunden lang bei 36 °C belassen. Nach Beendigung des Färbeprozesses wurde das Haar gespült, mit einem üblichen Haarwaschmittel gewaschen und anschließend getrocknet. Es resultierte eine naturbraune Farbe.

Erfindungsgemäße Färbemittel mit Ammonium- oder Metallsalz

Beispiel 3

Herstellung einer Färbelösung:

Es wurde eine Aufschlämmung von 1 mMol Isatin und 1 mMol Aminosäure bzw. Oligopeptid in 100 ml Wasser bereitet. Die erfindungsgemäßen Zusammensetzungen enthielten zusätzlich 1 mMol Ammonium- oder Metallsalz (Ausnahme: im Falle von $AlCl_3$ und Al-lactat wurde zwischen 0,05 und 1 mMol variiert). In einigen Fällen wurde der Färbelösung 1 mMol Natriumacetat (NaAc), Kaliumacetat (KAc) oder Na-glykolat zugesetzt.

Die Aufschlämmung wurde auf Siedetemperatur erhitzt und nach dem Abkühlen filtriert, der pH-Wert wurde anschließend auf 6 eingestellt. In diese Färbelösung wurden zu 90 % ergraute, nicht vorbehandelte Menschenhaare eingebracht. Die jeweiligen Färbetemperaturen, Färbezeiten, Farbnuancen und Farbtiefen sind Tabelle 1 zu entnehmen.

Die Farbtiefe wurde dabei nach folgender Skala bewertet:

| | |
|---|---|
| - : | keine oder eine sehr blasse Ausfärbung |
| (+) : | schwache Intensität |
| + : | mittlere Intensität |
| +(+) : | mittlere bis starke Intensität |
| ++ : | starke Intensität |
| ++(+) : | starke bis sehr starke Intensität |
| +++ : | sehr starke Intensität |

**Tabelle 2**

| Aminosäure (bzw. Oligopeptid) | Salze* | Färbetemperatur (in °C) | Färbezeit (in Stunden) | Färbenuance | Farbtiefe |
|---|---|---|---|---|---|
| | $(NH_4)_2CO_3$ NaAc | 40 | 2,0 | dunkelviolett | +++ |
| | $(NH_4)_2CO_3$ NaAc | 40 | 0,5 | violett | + |
| L-Prolin | $(NH_4)_2CO_3$ NaAc | 35 | 0,5 | violett | + |
| | $(NH_4)_2CO_3$ | 35 | 0,5 | violettrot | ++(+) |
| | $Sr(NO_3)_2$ NaAc | 35 | 0,5 | rotviolett | +(+) |
| | $MgCl_2$ NaAc | 35 | 0,5 | rotviolett | + |
| L-Arginin | $(NH_4)_2CO_3$ NaAc | 40 | 0,5 | violettrot | ++ |
| | $(NH_4)_2CO_3$ NaAc | 35 | 0,5 | violettrot | + |
| | $CaCl_2$ NaAc | 40 | 0,5 | rosarot | + |
| | $CaCl_2$ NaAc | 35 | 0,5 | orangerot | + |
| | $AlCl_3$ (0,5 mMol) | 40 | 2,0 | dunkelbraun | ++ |
| L-Tryptophan | $AlCl_3$ (0,5 mMol) | 35 | 0,5 | mittelrotbraun | + |
| | $AlCl_3$ (0,05 mMol) | 35 | 0,5 | mittelrotbraun | + |

**Fortsetzung Tabelle 2**

| Aminosäure (bzw. Oligopeptid) | Salze* | Färbetemperatur (in °C) | Färbezeit (in Stunden) | Färbenuance | Farbtiefe |
|---|---|---|---|---|---|
| L-Trytophan | $Sr(NO_3)_2$ NaAc | 40 | 0,5 | rotbraun | ++ |
| | $Sr(NO_3)_2$ NaAc | 35 | 0,5 | rotbraun | + |
| | $(NH_4)_2CO_3$ NaAc | 40 | 0,5 | schwarzviolett | +++ |
| | $(NH_4)_2CO_3$ NaAc | 40 | 0,5 | dunkelviolett | ++ |
| | $(NH_4)_2CO_3$ NaAc | 35 | 0,5 | braunorange | + |
| | Al-lactat NaAc (0,25 mMol) | 35 | 0,5 | mittelbraun | +(+) |
| | Al-lactat NaAc (0,05 mMol) | 35 | 0,5 | mittelbraun | +(+) |
| | Al-lactat | 35 | 0,5 | rotbraun | ++ |
| | Al-lactat KAc | 35 | 0,5 | mittelbraun | ++ |
| L-Tyrosin | $ZnCl_2$ | 35 | 0,5 | gelbgrün | (+) |
| | $ZnCl_2$ Na-glykolat | 35 | 0,5 | gelbgrün | + |
| | $ZnCl_2$ NaAc | 40 | 0,5 | kupfer | + |
| | $ZnCl_2$ NaAc | 35 | 0,5 | kupfer | +(+) |
| | $Sr(NO_3)_2$ NaAc | 40 | 0,5 | braunorange | + |
| | Al-lactat NaAc | 40 | 0,5 | gelbbraun | + |
| | $(NH_4)_2CO_3$ NaAc | 35 | 0,5 | olivgrün | + |
| L-Dopa | $ZnCl_2$ $NH_4Ac$ | 35 | 0,5 | braungelb | + |
| | $CaCl_2$ NaAc | 35 | 0,5 | braungelb | + |
| | $ZnCl_2$ NaAc | 35 | 0,5 | orangegelb | +(+) |
| | $(NH_4)_2CO_3$ NaAc | 35 | 0,5 | gelborangebraun | + |

* Wenn nicht anders angegeben, 1 mMol eines jeden aufgeführten Salzes pro 100 ml Wasser.

Beispiel 4

Folgendes Färbemittel wurde zur Anwendung auf dem Haar bereitet:

| | |
|---|---|
| Johannisbrotkernmehl | 2,0 g |
| Natriumacetat | 1,0 g |
| Ascorbinsäure | 1,0 g |
| Fettalkohol $C_{12/18}$-sulfat, Na-salz | 1,0 g |
| Isatin | 13 mMol |
| L-Prolin | 3,25 mMol |
| L-Tryptophan | 9,75 mMol |
| $Sr(NO_3)_2$ | 1,0 g |
| Wasser | ad 100 g |

Das Färbemittel wurde auf hellblondes Naturhaar aufgetragen und dort 2 Stunden bei 35 °C belassen. Nach Beendigung des Färbeprozesses wurde das Haar gespült, mit einem üblichen Haarwaschmittel gewaschen und anschließend getrocknet. Es resultierte eine naturbraune Nuance.

## Patentansprüche

1. Mittel zum Färben von keratinhaltigen Fasern, enthaltend mindestens ein Isatinderivat der Formel I,

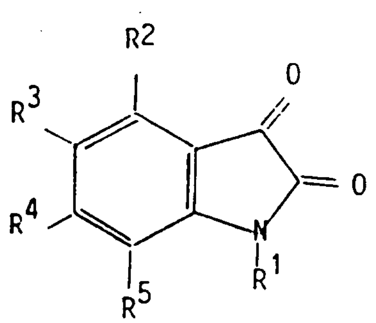

(I),

wobei $R^1$ Wasserstoff, eine Alkylgruppe mit 1 bis 4 C-Atomen, eine Hydroxyalkylgruppe mit 2 bis 4 C-Atomen, eine $C_2$-$C_{20}$-Acylgruppe, eine Phenylgruppe oder eine Benzoylgruppe und $R^2$, $R^3$, $R^4$ und $R^5$ unabhängig voneinander Wasserstoff, Hydroxy, Halogen, Nitrogruppen, Alkylgruppen mit 1 bis 4 C-Atomen, Alkoxygruppen mit 1 bis 4 C-Atomen oder $R^6R^7$N-Gruppen bedeuten, worin $R^6$ und $R^7$ unabhängig voneinander Wasserstoff, Alkylgruppen mit 1 bis 4 C-Atomen oder Hydroxyalkylgruppen mit 2 bis 4 C-Atomen darstellen und zwei benachbarte Gruppen $R^2$, $R^3$, $R^4$ und $R^5$ auch eine Alkylendioxygruppe mit 1 bis 4 C-Atomen darstellen können, und mindestens eine Aminosäure oder ein aus 2 bis 9 Aminosäuren aufgebautes wasserlösliches Oligopeptid in einem wasserhaltigen Träger.

2. Mittel zum Färben von keratinhaltigen Fasern nach Anspruch 1, dadurch gekennzeichnet, daß im Isatinderivat der Formel I $R^1$ Wasserstoff ist und $R^2$, $R^3$, $R^4$ und $R^5$ unabhängig voneinander Nasserstoff, Hydroxy, Halogen, Nitrogruppen oder $R^6R^7$N-Gruppen darstellen, worin $R^6$ und $R^7$ Wasserstoff bedeuten.

3. Mittel zum Färben von keratinhaltigen Fasern nach Anspruch 1 und 2, dadurch gekennzeichnet, daß die Verbindung der Formel I Isatin ist.

4. Mittel zum Färben von keratinhaltigen Fasern nach Anspruch 1 bis 3 enthaltend mindestens eine $\alpha$-Aminosäure, vorzugsweise aus der Gruppe Arginin, Cystein, Methionin, Prolin, Tyrosin, Valin, Glycin, Glutaminsäure, Histidin, Asparaginsäure, Alanin, Tryptophan, Cystin, Lysin, Hydroxyprolin, Leucin, Isoleucin, Phenylalanin, Serin, Ornithin, Threonin.

5. Mittel zum Färben von keratinhaltigen Fasern nach Anspruch 1 bis 4, enthaltend zusätzlich mindestens ein Salz ausgewählt aus der Gruppe der Ammonium-, Lithium-, Magnesium-, Calcium-, Strontium-, Barium-, Aluminium-, Titan-, Mangan-, Eisen-, Kobalt-, Nickel-, Kupfer-, Silber-, Zink-, Lanthan-, Cer-, Praseodym-, Neodym- und Gadoliniumsalze,

6. Mittel zum Färben von keratinhaltigen Fasern nach Anspruch 5, dadurch gekennzeichnet, daß die Salze Halogenide, Nitrate, Nitrite, Acetate, Lactate, Glykolate, Tartrate, Citrate, Gluconate, Propionate, Sulfate, Carbonate, Succinate, Phosphate, Phosphonate oder Sulfonate sind.

7. Mittel nach Anspruch 5 und 6, dadurch gekennzeichnet, daß die Salze ausgewählt sind aus der Gruppe Ammoniumcarbonat-, -chlorid und -acetat, Lithiumbromid, Magnesiumchlorid und -sulfat, Calciumchlorid und -gluconat, Strontiumchlorid und -nitrat, Aluminiumchlorid und -lactat, Zinkchlorid und -sulfat, Kupfer-(II)-chlorid und -sulfat und Lanthannitrat.

8. Haarfärbemittel enthaltend mindestens ein Isatin der Formel I in einer Menge von 0,3 bis 65, vorzugsweise 6 bis 20 mMol, mindestens eine Aminosäure oder ein aus 2 bis 9 Aminosäuren aufgebautes wasserlösliches Oligopeptid in einer Menge von 0,3 bis 65, vorzugsweise 6 bis 20 mMol, jeweils bezogen auf 100 g der gesamten Färbemittelzubereitung, und einen wasserhaltigen Träger.

9. Haarfärbemittel nach Anspruch 8, dadurch gekennzeichnet, daß zusätzlich mindestens ein Salz ausgewählt aus der Gruppe der Ammonium-, Lithium-, Magnesium-, Calcium-, Strontium-, Barium-, Aluminium-, Titan-, Mangan-, Eisen-, Kobalt-, Nickel-, Kupfer-, Silber-, Zink-, Lanthan-, Cer-, Praseodym-, Neodym- und Gadoliniumsalze, in einer Menge von 0,3 bis 65, vorzugsweise 2 bis 15 mMol, bezogen auf 100 g der gesamten Färbemittelzubereitung, enthalten ist.

## Claims

1. Formulations for colouring keratin fibres containing at least one isatin derivative corresponding to formula I:

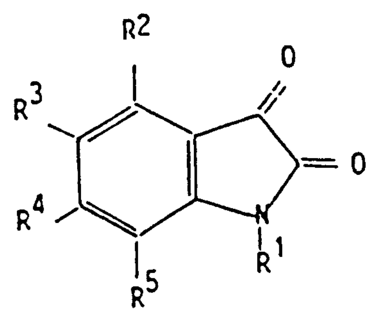

(I),

in which $R^1$ is hydrogen, a $C_{1-4}$ alkyl group, a $C_{2-4}$ hydroxyalkyl group, a $C_{2-20}$ acyl group, a phenyl group or a benzoyl group and $R^2$, $R^3$, $R^4$ and $R^5$ independently of one another represent hydrogen, hydroxy, halogen, nitro groups $C_{1-4}$ alkyl groups, $C_{1-4}$ alkoxy groups or $R^6R^7N$ groups where $R^6$ and $R^7$ independently of one another represent hydrogen, $C_{1-4}$ alkyl groups or $C_{2-4}$ hydroxyalkyl groups and two adjacent groups $R^2$, $R^3$, $R^4$ and $R^5$ may also represent a $C_{1-4}$ alkylenedioxy group,
and at least one amino acid or a water-soluble oligopeptide synthesized from 2 to 9 amino acids in a water-containing carrier.

2. Formulations for colouring keratin fibres as claimed in claim 1, characterized in that, in the isatin derivative corresponding to formula I, $R^1$ is hydrogen and $R^2$, $R^3$, $R^4$ and $R^5$ independently of one another represent hydrogen, hydroxy, halogen, nitro groups or $R^6R^7N$ groups where $R^6$ and $R^7$ represent hydrogen.

3. Formulations for colouring keratin-containing fibres as claimed in claims 1 and 2, characterized in that the compound corresponding to formula I is isatin.

4. Formulations for colouring keratin-containing fibres as claimed in claims 1 to 3 containing at least one $\alpha$-amino acid preferably from the group consisting of arginine, cysteine, methionine, proline, tyrosine, valine, glycin, glutamic acid, histidine, aspartic acid, alanine, tryptophan, cystine, lysine, hydroxyproline, leucine, isoleucine, phenyl alanine, serine, ornithine, threonine.

5. Formulations for colouring keratin-containing fibres as claimed in claims 1 to 4 additionally containing at least one salt selected from the group of ammonium, lithium, magnesium, calcium, strontium, barium, aluminium, titanium, manganese, iron, cobalt, nickel, copper, silver, zinc, lanthanum, cerium, praseodymium, neodymium and gadolinium salts.

6. Formulations for colouring keratin-containing fibres as claimed in claim 5, characterized in that the salts are halides, nitrates, nitrites, acetates, lactates, glycolates, tartrates, citrates, gluconates, propionates, sulfates, carbonates, succinates, phosphates, phosphonates or sulfonates.

7. Formulations as claimed in claims 5 and 6, characterized in that the salts are selected from the group consisting of ammonium carbonate, chloride and acetate, lithium bromide, magnesium chloride and sulfate, calcium chloride and gluconate, strontium chloride and nitrate, aluminium chloride and lactate, zinc chloride and sulfate, copper(II) chloride and sulfate and lanthanum nitrate.

8. Hair colouring formulations containing at least one isatin corresponding to formula I in a quantity of 0.3 to 65 and preferably 6 to 20 mmoles, at least one amino acid or a water-soluble oligopeptide synthesized from 2 to 9 amino acids in a quantity of 0.3 to 65 and preferably 6 to 20 mmoles, based in both cases on 100 g of the colouring formulation as a whole, and a water-containing carrier.

9. Hair colouring formulations as claimed in claim 8, characterized in that at least one salt selected from the group of ammonium, lithium, magnesium, calcium, strontium, barium, aluminium, titanium, manganese, iron, cobalt, nickel, copper, silver, zinc, lanthanum, cerium, praseodymium, neodymium and gadolinium salts is additionally present in a quantity of 0.3 to 65 and preferably 2 to 15 mmoles, based on 100 g of the colouring formulation as a whole.

## Revendications

1. Agents pour la coloration de fibres kératiniques renfermant au moins un dérivé de l'isatine de formule I :

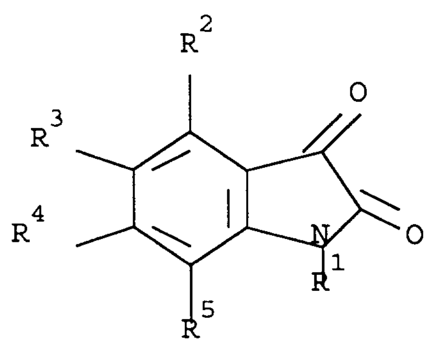

(I),

dans laquelle $R^1$ signifie de l'hydrogène, un groupe alcoyle ayant de 1 à 4 atomes de carbone, un groupe hydroxyalcoyle ayant de 2 à 4 atomes de carbone, un groupe acyle en $C_2$ à $C_{20}$, un groupe phényle, ou un groupe benzoyle, et $R^2$, $R^3$, et $R^5$ indépendamment l'un de l'autre, représentent de l'hydrogène, un hydroxy, un halogène, des groupes nitro, des groupes alcoyle ayant de 1 à 4 atomes de carbone, des radicaux alcoxy ayant de 1 à 4 atomes de carbone ou des groupes $R^6R^7N$ où $R^6$ et $R^7$ indépendamment l'un de l'autre représentent de l'hydrogène, des groupes alcoyle ayant de 1 à 4 atomes de carbone, ou des groupes hydroxyalcoyle ayant de 2 à 4 atomes de carbone, et deux groupes voisins $R^2$, $R^3$ $R^4$ et $R^5$ peuvent représenter aussi un groupe alcoylènedioxy ayant de 1 à 4 atomes de carbone ainsi qu'un aminoacide ou un oligopeptide soluble dans l'eau, constitué de 2 à 9 aminoacides dans un support contenant de l'eau.

**2.** Agent pour la coloration de fibres kératiniques selon la revendication 1, caractérisé en ce que dans le dérivé d'isatine de formule I, $R^1$ est de l'hydrogène et $R^2$, $R^3$, $R^4$ et $R^5$, indépendamment l'un de l'autre signifient de l'hydrogène, un hydroxy, un halogène, des groupes nitro, ou des groupes $R^6R^7N$ dans lesquels $R^6$ et $R^7$ signifient de l'hydrogène.

**3.** Agent pour la coloration des fibres kératiniques selon les revendications 1 et 2, caractérisé en ce que le composé de formule I est l'isatine.

**4.** Agent pour la coloration des fibres kératiniques selon les revendications 1 à 3 qui renferme au moins un α-aminoacide, de préférence choisi dans le groupe de l'arginine, de la cystéine, de la méthionine, de la proline, de la tyrosine, de la valine, de la glycine, de l'acide glutamique, de l'histidine, de l'acide aspartique, de l'alanine, du tryptophane, de la cystine, de la lysine, de l'hydroxyproline, de la leucine, de l'isoleucine, de la phénylalanine, de la serine, de l'ornithine, de la thréonine.

**5.** Agent pour la coloration des fibres kératiniques selon les revendications 1 à 4, qui renferme en supplément au moins un sel choisi dans le groupe des sels d'ammonium, de lithium, de magnésium, de calcium, de strontium, de baryum, d'aluminium, de titane, de manganèse, de fer, de cobalt, de nickel, de cuivre, d'argent, de zinc, de lanthane, de cérium, de praséodyme, de neodyme, et de gadolinium.

**6.** Agent pour la coloration des fibres kératiniques selon la revendication 5, caractérisé en ce que les sels sont des halogénures, des nitrates, des nitrites, des acétates, des lactates, des glycolates, des tartrates, des citrates, des gluconates, des propionates, des sulfates, des carbonates, des succinates, des phosphates, des phosphonates ou des sulfonates.

**7.** Agent selon les revendications 5 et 6, caractérisé en ce que les sels sont choisis dans le groupe du carbonate, du chlorure et de l'acétate d'ammonium, du bromure de lithium, du chlorure et du sulfate de magnésium, du chlorure et du gluconate de calcium, du chlorure et du nitrate de strontium, du chlorure et du lactate d'aluminium, du chlorure et du sulfate de zinc, du chlorure et du sulfate de cuivre (II) et du nitrate de lanthane.

**8.** Teinture pour les cheveux qui renferme au moins une isatine de formule I en quantités allant de 0,3 à 65, de préférence de 6 à 20 mMol, au moins un aminoacide ou un oligopeptide soluble dans l'eau constitué de 2 à 9 aminoacides en quantités allant de 0,3 à 65, de préférence de 6 à 20 mMol, à chaque fois rapporté à 100 g de la préparation totale de teinture, et un support contenant de l'eau.

**9.** Teinture pour les cheveux selon la revendication 8, caractérisée en ce qu'en supplément on y inclut au moins un sel choisi dans le groupe des sels d'ammonium, de lithium, de magnésium, de calcium, de strontium, de baryum, d'aluminium, de titane, de manganèse, de fer, de cobalt, de nickel, de cuivre, d'argent, de zinc, de lanthane, de cérium, de praséodyme, de neodyme, et de gadolinium, en quantités allant de 0,3 à 65 de préférence de 2 à 15 mMol, rapporté à 100 g de la préparation totale de teinture.